# EUROPEAN PATENT SPECIFICATION

(11) **EP 3 810 038 B1**
(45) Date of publication and mention of the grant of the patent: **24.04.2024**
(21) Application number: 19735161.2
(22) Date of filing: 19.06.2019
(51) Int. Cl.: A61F 2/24, A61F 2/915

(54) **SUPPORT STRUCTURE FOR AN IMPLANTABLE DEVICE WITH ENHANCED COMPRESSIVE STIFFNESS REGION(S)**
TRÄGERSTRUKTUR FÜR EINE IMPLANTIERBARE VORRICHTUNG MIT VERBESSERTEN DRUCKSTEIFIGKEITSREGIONEN
STRUCTURE DE SUPPORT POUR UN DISPOSITIF IMPLANTABLE COMPRENANT UNE OU PLUSIEURS RÉGIONS À RIGIDITÉ À LA COMPRESSION AMÉLIORÉE

(30) Priority: 20.06.2018 US 201862687704 P; 26.04.2019 US 201962839164 P
(43) Date of publication of application: 28.04.2021
(73) Proprietor: W. L. Gore & Associates, Inc., Newark, DE 19711 (US)
(72) Inventor: DIENNO, Dustin V., Newark, DE 19711 (US)
(74) Representative: HGF
(86) International application number: PCT/US2019/037998
(87) International publication number: WO 2019/246268

(56) References cited:
- EP-A1- 1 557 138
- WO-A1-97/04721
- US-A1- 2007 233 232
- US-A1- 2008 154 355
- US-A1- 2009 234 429
- US-A1- 2013 211 498
- US-A1- 2016 262 878
- US-A1- 2017 360 585

## Description

### CROSS-REFERENCE TO RELATED APPLICATION

This application claims the benefit of Provisional Application No. 62/839,164, filed April 26, 2019, and also claims the benefit of Provisional Application No. 62/687,704, filed June 20, 2018.

### FIELD

The present disclosure relates to implantable medical devices including prosthetic valves, and support structures for implantable medical devices including expandable support structures for prosthetic heart valves.

### BACKGROUND

Various types of support structures are implemented in association with implantable medical devices. Self-expanding nitinol frames are often used as a support structure for replacement heart valves. These devices can be deployed into a calcified aortic annulus for treatment. In various circumstances, such support structures either have insufficient flat plate stiffness to perform effectively or exert undesirable, chronic radial forces on the surrounding tissue.

US2008/154355A1 discloses a support structure for a prosthetic heart valve.

### SUMMARY

Various examples relate to support structures (e.g., prosthetic valve structures) that incorporate a frame that, upon transitioning to a deployed configuration, include a proximal section has increased stiffness, or resistance to deformation in a transverse plane to a longitudinal axis of a device, including resistance to a change in shape, size, or both. Such an increase in transverse deformation resistance may be measured as an increase in radial compressive resistance or an increase in flat plate stiffness, for example, or both. Such increases in transverse deformation resistance may be realized through a reduction in length of the increased stiffness region of the support structure, such as through longitudinal compression of the region following an initial radial expansion of the region.

The invention is defined by the appended claims.

According to an example ("Example 1") a support structure for an implantable device includes a tubular body having a longitudinal axis, the tubular body including a first region that is annular shape and is characterized by a first transverse deformation resistance (e.g., a first radial compressive resistance and/or first flat plate stiffness); and a second region that is annular in shape and is characterized by a second transverse deformation resistance (e.g., a second radial compressive resistance and/or second flat plate stiffness). The second region includes a plurality of strut elements that define the annular shape of the second region. The plurality of strut elements may have a shape / geometry (e.g., curvature) and / or cross-section such that the second region exhibits a relatively higher degree of compressibility in the longitudinal direction than in the radial or transverse direction. According to the invention, at least a portion of each of the plurality of strut elements have a region of reduced cross section.

At least a portion of each of the plurality of strut elements has a region of reduced cross section including a width in a radial direction transverse to the longitudinal axis of the tubular body and a thickness in a longitudinal direction parallel to the longitudinal axis of the tubular body, the width in the region of reduced cross section being greater than the thickness (e.g., at least four times, six times or some other multiple the thickness) such that the second region exhibits a relatively higher degree of compressibility in the longitudinal direction than in the radial direction.

The second transverse deformation resistance (e.g., radial and/or flat plate stiffness) may be greater than the first transverse deformation resistance (e.g., radial and/or flat plate stiffness).

The first region may include a plurality of strut elements defining the annular shape of the first region, and further wherein at least a portion of each of the plurality of strut elements of the first region has a width in the radial direction transverse to the longitudinal axis of the tubular body and a thickness in the longitudinal direction parallel to the longitudinal axis of the tubular body, the width of each of the plurality of strut elements of the first region being less than 4 times the thickness of each of the plurality of strut elements of the first region.

The support structure may include one or more leaflets coupled to the first region.

The one or more leaflets may be formed of a natural material.

The one or more leaflets may be formed of a synthetic material.

According to another example ("Example 7") not according to the invention, a support structure for an implantable device includes a tubular body having a longitudinal axis, the implantable device being transitionable between a delivery configuration and a deployed configuration, and the tubular body includes a first region that is annular shape and is characterized by a first transverse deformation resistance; and a second region that is annular in shape and is characterized by a second transverse deformation resistance, the second region including a plurality of strut elements defining the annular shape of the second region, the plurality of strut elements intersecting with one another to form a plurality of cells, each of the plurality of cells defining a longitudinal apex facing a longitudinal direction along the longitudinal axis of the support structure and a lateral apex transverse to the longitudinal apex and facing along a circumference of the support structure, and further wherein in the deployed configuration the longitudinal apex defines an obtuse angle and the circumferential apex defines an acute angle.

Each of the plurality of cells may define a pair of longitudinal apices facing the longitudinal direction and a pair of lateral apices facing along the circumferential direction, and further wherein in the deployed configuration each of the longitudinal apices defines an obtuse angle and each of the lateral apices defines an acute angle.

Each pair of lateral apices of the plurality of cells may define a circumferential centerline of the plurality of cells that extends between the pair of lateral apices of each of the plurality of cells, and further wherein at least a portion of each of the intersecting frame members define the pair of longitudinal apices of each of the plurality of cells intersecting the circumferential centerline of each of the plurality of cells.

The obtuse angle defined by each longitudinal apex in the deployed configuration may be greater than 100 degrees, 130 degrees, 150 degrees, or 170 degrees, or any value or range between the foregoing values.

The obtuse angle defined by each longitudinal axis may be between 100 degrees and 170 degrees.

The second transverse deformation resistance may be greater than the first transverse deformation resistance.

According to another example not according to the invention, a method of implanting a prosthetic valve includes advancing the prosthetic valve to a target region within a patient's anatomy in a delivery configuration in which the prosthetic valve is at a first, compressed delivery diameter, the prosthetic valve including a support structure comprising a first region having an annular shape and characterized by a first transverse deformation resistance, and a second region having an annular shape and characterized by a second transverse deformation resistance, wherein the second region includes a plurality of strut elements intersecting with one another to form a plurality of cells, each of the plurality of cells defining a longitudinal apex facing a longitudinal direction along the longitudinal axis of the support structure and a lateral apex transverse to the longitudinal apex and facing along a circumference of the support structure, wherein when the prosthetic valve is in the delivery configuration the longitudinal apex defines an acute angle and the circumferential apex defines an obtuse angle; and deploying the prosthetic valve such that the longitudinal apex defines an obtuse angle and the circumferential apex defines an acute angle. In some implementations, deploying the prosthetic valve further includes axially, or longitudinally compressing the second region and locking the second region in an axially compressed configuration.

The obtuse angle of the longitudinal apex may be at least one hundred eighty (180) degrees.

The obtuse angle of the longitudinal apex may exceed one hundred eighty (180) degrees.

Also disclosed herein is , a method of forming a support structure for a prosthetic valve includes cutting a pattern of closed cells from a tube to form the support structure having a first diameter and a first length, each closed cell being defined by a plurality of fame members, expanding the first diameter of the support structure from the first diameter to a second diameter, axially compressing a portion of less than all of the length of the support structure to form a first region and a second region, the first region including a first plurality of cells and the second region including a second plurality of cells, wherein a shape of the cells in the second plurality of cells differs from a shape of the cells in the first plurality of cells, heat setting the support structure at a geometry having the first and second regions such that the first region is characterized by a first transverse deformation resistance, and such that the second region is characterized by a second transverse deformation resistance different from the first transverse deformation resistance.

The cells of the support structure each may have the same shape (e.g., each being generally diamond-shaped) prior to axially compressing the portion of less than all of the length of the support structure to form the first region and the second region. In various implementations, the cells of the second region have a relatively more elongate shape in the longitudinal direction than the transverse direction in an initial, delivery configuration.

A support structure of an implantable device may include a framework having a first region configured to support one or more leaflets and a second region configured to provide enhanced transverse deformation resistance, the second region defining a plurality cells each having a first height and width when the second region is at a delivery configuration, a second height and width when the second is expanded from the delivery configuration to an initial, expanded configuration, and a third height and width when the second region is longitudinally compressed from the initial, expanded configuration to a final, deployed configuration having enhanced transverse deformation resistance relative to the initial, expanded configuration, the support structure including at least one locking mechanism configured to lock one or more of the plurality of cells at the third height and width.

The locking mechanism optionally includes a first lock component projecting within a corresponding cell, the first lock component including a first sliding surface and a first projection and defining a first receiver; and a second lock component projecting toward the first lock component, the second lock component including a second sliding surface and a second projection and defining a second receiver.

In some examples, the first and second lock components are configured such that the first and second sliding surfaces slide against each during collapse of the corresponding cell to facilitate receipt of the first projection in the second receiver and the second projection in the first receiver to lock the locking mechanism and retain the corresponding cell in a collapsed configuration.

The support structure is included as part of a prosthetic valve.

The support structure is a stent structure.

The optional first and second sliding surfaces may cause the first and second locking components to elastically deflect during collapse of the corresponding cell.

The optional first and second locking components may be symmetric in shape.

The optional first and second locking components may be integrally formed with the support structure.

The foregoing Examples are just that, and should not be read to limit or otherwise narrow the scope of any of the inventive concepts otherwise provided by the instant disclosure. While multiple examples are disclosed, still other embodiments will become apparent to those skilled in the art from the following detailed description, which shows and describes illustrative examples. Accordingly, the drawings and detailed description are to be regarded as illustrative in nature rather than restrictive in nature.

### BRIEF DESCRIPTION OF THE DRAWINGS

The accompanying drawings are included to provide a further understanding of inventive embodiments of the disclosure and are incorporated in and constitute a part of this specification, illustrate examples, and together with the description serve to explain various inventive principles.
Figure 1 shows a prosthetic valve including a support structure having a first, leaflet attachment region and a second region of increased transverse deformation resistance, according to some embodiments.
Figure 2 shows the support structure of the prosthetic valve of Figure 1 with the leaflets and the graft material removed, according to some embodiments.
Figure 3 shows a first, leaflet attachment region of a prosthetic valve, according to some embodiments.
Figure 4 shows a second region of increased transverse deformation resistance of the prosthetic valve of Figure 1, according to some embodiments.
Figures 5A to 5C show various stages of deployment of a prosthetic valve including a support structure, with the leaflets and the graft material removed, according to some embodiments.
Figure 6 shows a second region of a support structure of a prosthetic valve in configuration corresponding to an initial state for delivery during implantation, according to some embodiments.
Figures 7A to 7C show a row of structural elements of the region of increased transverse deformation resistance of the prosthetic valve of Figure 1 in an initial configuration, according to some embodiments.
Figure 8A to 8C shows detailed views of a cell within the rows of structural elements shown in Figures 7A to 7C, according to some embodiments.
Figure 9 shows a locking mechanism, according to some embodiments not according to the invention.

### DEFINITIONS AND TERMINOLOGY

This disclosure is not meant to be read in a restrictive manner. For example, the terminology used in the application should be read broadly in the context of the meaning those in the field would attribute such terminology.

With respect to terminology of inexactitude, the terms "about" and "approximately" may be used, interchangeably, to refer to a measurement that includes the stated measurement and that also includes any measurements that are reasonably close to the stated measurement. Measurements that are reasonably close to the stated measurement deviate from the stated measurement by a reasonably small amount as understood and readily ascertained by individuals having ordinary skill in the relevant arts. Such deviations may be attributable to measurement error, differences in measurement and/or manufacturing equipment calibration, human error in reading and/or setting measurements, minor adjustments made to optimize performance and/or structural parameters in view of differences in measurements associated with other components, particular implementation scenarios, imprecise adjustment and/or manipulation of objects by a person or machine, and/or the like, for example. In the event it is determined that individuals having ordinary skill in the relevant arts would not readily ascertain values for such reasonably small differences, the terms "about" and "approximately" can be understood to mean plus or minus 10% of the stated value.

The term "transverse deformation resistance" as used herein refers to resistance to deformation in a substantially transverse plane to a longitudinal axis of a support structure. Examples of measures of transverse compressive resistance include radial compressive resistance and flat plate stiffness, for example.

### DETAILED DESCRIPTION

Persons skilled in the art will readily appreciate that the various embodiments of the inventive concepts provided in the present disclosure can be realized by any number of methods and apparatuses configured to perform the intended functions. It should also be noted that the accompanying figures referred to herein are not necessarily drawn to scale, but may be exaggerated to illustrate various aspects of the present disclosure, and in that regard, the drawing figures should not be construed as limiting.

Various examples address expandable support structures with one or more regions of enhanced transverse deformation resistance (e.g., enhanced flat plate stiffness and/or radial compressive resistance). Various examples include configurations that help minimize the disadvantages associated with high degrees of chronic outward radial force. Support structures having enhanced transverse deformation resistance can be advantageous for promoting and/or maintaining the reshaping of the tissue structures into which they are implanted. For example, where an expandable support structure consistent with various examples discussed herein is employed in a prosthetic valve implanted in a valve orifice, it may be desirable for the prosthetic valve to stent open the valve orifice and re-circularize the annulus so that performance of the leaflets of the prosthetic valve is not hindered, thus providing a support structure that helps maximize the capability of the leaflets to operate at their kinematically-optimal deployed shape.

In various examples, the enhanced transverse deformation resistance regions are fully radially expanded by longitudinally compressing them. The enhanced transverse deformation resistance regions can be first expanded to an intermediate deployed diameter, and then longitudinally compressed to a fully deployed diameter at which the regions exhibit their desired transverse deformation resistance. Such expansion techniques and associated support structure configurations facilitate expansion to a known/pre-selected diameter which can help reduce wrinkles/deformations in leaflet shape following deployment that would otherwise lead to suboptimal leaflet kinematics. Such expansion mechanisms and enhanced transverse deformation resistance can also help ensure uniform cell expansion/configuration and more consistent performance (e.g., fatigue resistance, deployed shape, stiffness, and the like) following deployment.

The transverse deformation resistance (e.g., radial compressive resistance and/or flat plate stiffness) of many conventional self-expanding prosthetic support structure designs may not be sufficiently high to overcome the stiffness/resistance of surrounding tissue (e.g., tissue of a calcified valve annulus) to reshape or to otherwise maintain a desired shape of the surrounding tissue. Comparatively, expandable prosthetic support structure designs (e.g., expandable or self-expanding) that have a high outward radial force to overcompensate for relatively lower transverse deformation resistance (e.g., oversized self-expanding structures), may exert chronic outward forces on surrounding tissue that may give rise to other issues, including conduction system disturbance issues (e.g., bundle branch block, complete heart block, and the need for pacemaker implantation).

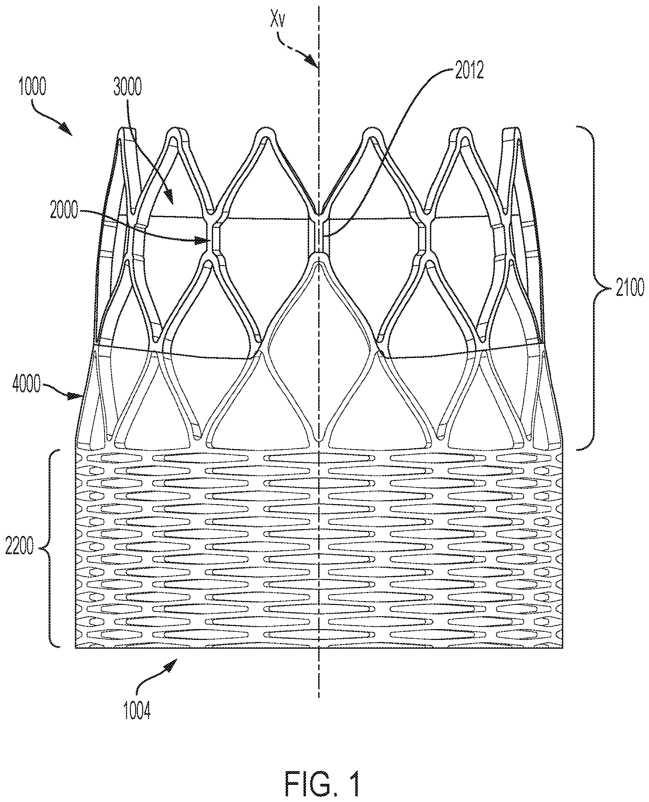

FIG. 1 shows prosthetic valve 1000 including a support structure 2000 and one or more leaflets 3000. In some examples, the prosthetic valve 1000 may be implemented as a blood valve, including a heart valve for example. In some examples, the prosthetic valve 1000 includes a graft material 4000. The graft material may be disposed about the prosthetic valve 1000 interior or exterior to the support structure 2000. The graft material 4000 may include any known biocompatible materials, including but not limited to polytetrafluoroethylene (PTFE), expanded polytetrafluoroethylene (ePTFE), or other suitable polymers or natural materials.

As shown in FIG. 1, the support structure 2000 includes a first portion or region 2100 and a second portion or region 2200. In various examples, the first portion or region 2100 is configured to house, accommodate, or support one or more leaflets 3000. The first portion or region 2100 (also referred to herein as a leaflet support portion) may define or otherwise reside at the distal end or distal end region 1002 of the prosthetic valve 1000. In some examples, the distal end or distal end region 1002 corresponds to an outflow portion of the prosthetic valve 1000. That is, in various examples, blood or fluid entering the prosthetic valve 1000 may enter or first encounter the prosthetic valve 1000 at a proximal end or end region 1004 (also referred to herein as an inflow region) of the prosthetic valve 1000. The fluid or blood generally travels through the prosthetic valve 1000 and exits the prosthetic valve 1000 at the distal end or distal end region 1002 of the prosthetic valve 1000 (also referred to herein as an outflow region). When traveling through the prosthetic valve 1000, the fluid or blood encounters the leaflets 3000, which control a direction of flow through the prosthetic valve 1000.

As described above, in various embodiments, one or more leaflets 3000 are coupled to the first region 2100, which can also be described as leaflet frame 2100, to provide a one-way valve structure. Any of a variety of mechanical valve, biological leaflet, and synthetic leaflet designs may be employed as desired. Examples of suitable leaflet constructions and methods of attachment to leaflet frame subcomponents are illustrated and described in U.S. Patent Application Nos. 13/833,650, 14/973,589, and 14/622,599. In general terms, the leaflets 3000 are coupled to the first region 2100 such that they are operable to open to allow flow from the inflow region to pass through the leaflet frame subcomponent outflow region, also referred to as the forward flow direction, and are operable to close to restrict flow from flowing from the outflow region through the inflow region, also referred to as the retrograde flow direction. The one or more leaflets 3000 can be coupled to the inner surface and/or outer surface of the support structure 2000, to a film that associated with the support structure 2000, and/or may be wrapped about one or more portions of the support structure 2000, for example, and a variety of suitable attachment mechanisms, including mechanical fasteners are also contemplated.

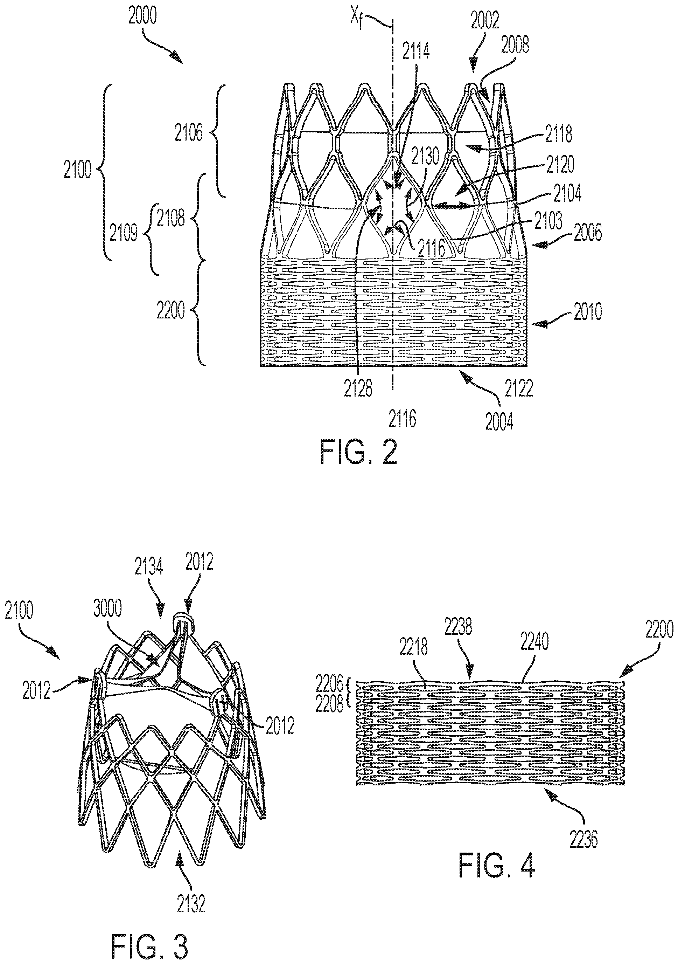

In some embodiments, the prosthetic valve 1000 has a central longitudinal axis Xv. Likewise, a central longitudinal axis Xf of the support structure 2000 is coaxial with the central axis Xv of the prosthetic valve 1000, as shown in FIG. 2, and may be described interchangeably with the central longitudinal axis Xv of the prosthetic valve 1000. With continued reference to FIG. 2, in various examples, the support structure 2000 includes a distal end 2002, a proximal end 2004, and an intermediate region 2006 defined between the proximal and distal ends 2002 and 2004. In some examples, the distal end 2002 of the support structure 2000 defines or otherwise corresponds with the distal end 1002 of the prosthetic valve 1000. Similarly, in some examples, the proximal end 2004 of the support structure 2000 defines or otherwise corresponds with the proximal end 1004 of the prosthetic valve 1000. As mentioned above, in various examples, the support structure 2000 includes a first region 2100 and a second region 2200, wherein the second region 2200 is generally configured to include an enhanced transverse deformation resistance. In some examples, the transverse deformation resistance of the second region 2200 is enhanced relative to the first region 2100. Put differently, in some examples, the first region 2100 includes a first transverse deformation resistance (e.g., radial compressive resistance and/or flat plate resistance), while the second region 2200 includes second transverse deformation resistance (e.g., radial compressive resistance and/or flat plate stiffness) that is greater than that of the first region 2100.

The prosthetic valve 1000, and thus the support structure 2000 along with the one or more leaflets 3000 and graft 4000, is optionally collapsible to a reduced profile, delivery configuration and then expandable (e.g., self-expanding or expanded by the application of an internal force, such as by balloon expansion or other expansion mechanism discussed herein) in situ. As shown in FIG. 2, the support structure 2000 is optionally annular, and may define an at least partially tapered cylinder (e.g., a cone), also described as a tapered cylindrical shape, though it is to be appreciated that the support structure 2000 may be entirely conically shaped or not conically shaped (e.g., a non-tapered constant cross section, such as a right angle cylinder). Where conically shaped, the support structure 2000 may include a relatively constant (linear) taper, although non-constant tapers (e.g., varies with one or more curved or angled segments) are contemplated.

Although the support structure 2000 generally defines a circular transverse cross-section in an unloaded state (e.g., when not under a transverse load), it should be understood that any variety of cross-sections (e.g., oval- or rectangular-shaped) are also contemplated. The support structure 2000 has an inner side 2008 and an outer side 2010 opposite the inner side 2008. The inner side 2008 faces toward the central longitudinal axis Xf, and the outer side 2010 faces outwardly, or away from the central longitudinal axis Xf. The support structure 2000 generally extends from the distal end 2002 (also described as an outflow end) to the proximal end 2004 (also described as an inflow end). As shown in FIG. 1, the support structure 2000 may include one or more commissure posts 2012 configured to interface with and provide support for the one or more leaflets 3000.

FIG. 3 shows another configuration of the first region 2100 of the support structure 2200 that may be combined with the second region 2200 (e.g., integrally formed with or coupled to). As shown, the support structure 2000 according to FIG. 3 may include one or more commissure posts 2012 that project distally from a remainder of the support structure and are configured to interface with and provide support for the one or more leaflets 3000. Although three commissure posts 2012 are shown in FIG. 3, any number of commissure posts are contemplated. The plurality of commissure posts 2012 define circumferentially-adjacent ones, or simply adjacent ones of the plurality of commissure posts 2012 moving about the perimeter of the support structure 2000.

In some examples, the transverse deformation resistance of the second region 2200 is enhanced relative to the transverse deformation resistance of the first region 2100 by way of one or more material properties of the various structural components of the second region 2200 (e.g., via selective strain hardening). In some examples, the transverse deformation resistance of the second region 2200 is additionally or alternatively enhanced relative to the transverse deformation resistance of the first region 2100 by why of the orientation and/or configuration of the various structural elements of the second region 2200.

For instance, as shown in FIG. 2, the first region 2100 of the support structure 2000 is comprised of a plurality of frame members including one or more strut elements 2102. The strut elements 2102 may be coupled together or may be configured to interface at one or more interface regions 2104 (also referred to herein as intersection locations Pd). The strut elements 2102 include a plurality of leaflet attachment struts 2103 to which one or more leaflets are able to be coupled.

In some embodiments, the plurality of strut elements 2102 define a collapsible (e.g., elastically) and expandable (e.g., self-expanding, or expandable via balloon or other suitable mechanism as discussed herein) framework, and also serve to support one or more leaflets, as mentioned above. As shown in FIGS. 2 and 3, the plurality of strut elements 2102 define a plurality of rows of closed cells 2118 (e.g., row 2106, row 2108, and row 2109) defining an alternating pattern of proximal-facing apices 2110 pointing in a proximal direction and distal-facing apices 2112 pointing in a distal direction. As shown in FIGS. 2 and 3, there are two rows of closed cells 2118, with a third row, row 2109, having a relatively flatter set of distal facing apices 2112. Although three are shown, greater or fewer numbers are contemplated (e.g., 1, 2, 4, 12, or 20).

In various examples, the apex angles 2114 of each of the distal-facing apices 2110 may have a value that is approximately the same in more than one of the plurality of rows (e.g., 2106 and 2108) of closed cells 2118, or values that vary as desired. In some embodiments, each of the apex angles 2114 of the distal-facing apices 2110 that are defined in rows 2106 and 2108 is within 10% of a common apex angle defined by those distal-facing apices 2110. In particular, it may be advantageous for the distal-facing apices 2110 that fall in the region where the leaflet(s) are attached to the support structure to approach a common apex angle. For example, the distal-facing apices 2110 including the leaflet attachment struts 2103 may approach a common apex angle, or otherwise be within a desired range of a common apex angle. Although a 10% range is given, in other embodiments, each of those apex angles is within 5%, 15%, 20%, or some other value of a common apex angle. In some examples, the common apex angle described above is 30 degrees, although any of a variety of common apex angles is contemplated (e.g., 10, 15, 20, 30, 40, 45, 50, 60, 90 degrees and ranges between any of those vales).

In various examples, the apex angles 2116 of each of the proximal-facing apices 2112 may have a value that is approximately the same in more than one of the plurality of rows (e.g., 2108 and 2109) of closed cells 2118, or values that vary as desired. In some embodiments, each of the apex angles 2116 of the proximally-facing apices 2112 that are defined in rows 2108 and 2109 is within 10% of a common apex angle defined by those proximal-facing apices 2112. In particular, it may be advantageous for the proximal-facing apices 2112 that fall in the region where the leaflet(s) are attached to the support structure to approach a common apex angle. For example, the proximal-facing apices 2112 including the leaflet attachment struts 2103 may approach a common apex angle, or otherwise be within a desired range of a common apex angle. Although a 10% range is given, in other embodiments, each of those apex angles is within 5%, 15%, 20%, or some other value of a common apex angle. In some examples, the common apex angle described above is 30 degrees, although any of a variety of common apex angles is contemplated (e.g., 10, 15, 20, 30, 40, 45, 50, 60, 90 degrees and ranges between any of those vales).

In some examples, the apex angles 2114 and/or the apex angles 2116 of one or more columns of closed cells 2118 defined by the plurality of closed cells 2118 are approximately the same as another one of the columns of closed cells 2118. For example, the apex angles of one or more columns is optionally within 10% of a common apex angle defined by the one or more columns of closed cells 2118 of proximal-facing apices 2112 and/or distal-facing apices 2110. In particular, it may be advantageous for the apices that fall in the region where the leaflet(s) are attached to the support structure to approach a common apex angle. For example, the apices including the leaflet attachment struts may approach a common apex angle, or otherwise be within a desired range of a common apex angle. Although a 10% range is given, in other embodiments, each of the apex angles is within 5%, 15%, 20%, or some other value of a common apex angle. In some examples, the common apex angle is 30 degrees, although any of a variety of common apex angles is contemplated (e.g., 10, 15, 20, 30, 40, 45, 50, 60, 90 degrees and ranges between any of those values).

The closed cells 2118 of the first region 2100 of the support structure 2000 generally intersect with one another at intersection locations Pd. As shown in the example of FIG. 4, each closed cell 2118 of the first region 2100 of the support structure 2000 has a cell height 2120 a cell width 2122, wherein the cell width 2122 can be generally understood to be perpendicular to the cell height 2120. Moreover, each closed cell 2118 has a first lateral-facing apex 2124 defining an apex angle 2128 and a second lateral facing-apex 2126 opposite the first lateral-facing apex 2124 and defining an apex angle 2130.

In a similar manner to the apex angles 2114 and the apex angles 2116, in various examples, one or more of the apex angles 2128 and/or apex angles 2130 has approximately the same value between one or more of the plurality of closed cells 2118 (e.g., within 10% of a common apex angle, although other values such as values within 5%, 15%, 20%, or some other value of a common apex angle are contemplated). In particular, and as mentioned above, it may be advantageous for the apices that fall in the region where the leaflet(s) are attached to the support structure to approach a common apex angle. For example, the apices including the leaflet attachment struts may approach a common apex angle, or otherwise be within a desired range of a common apex angle. In some examples, the common apex angle is 30 degrees, although any of a variety of common apex angles is contemplated (e.g., 10, 15, 20, 30, 40, 45, 50, 60, 90 degrees and ranges between any of those values).

In various examples, the cell heights 2120 of the closed cells 2118 may be the same or different for different rows and/or columns of closed cells 2118 of the first region 2100 of the support structure 2000. Similarly, the cell widths 2122 of the closed cells 2118 may be the same or different for different rows and/or columns of closed cells 2118 of the first region 2100 of the support structure 2000.

The In various of examples, varying the apex angles of the proximal and distal facing apices 2110 and 2112 and/or the lateral-facing apices 2124 and 2126 of the closed cells helps control compaction forces (e.g., the force needed for diametrically compacting the prosthetic valve 1000 to a compact delivery configuration) as well as the transverse deformation resistance of the particular region of the support structure 2000 upon deployment. It should also be understood that other factors, including material selection, strut geometry (cross-section and curvature), and other characteristics may be selected to vary transverse deformation resistance of the first region 2100. Thus, in various examples, the structural support 2000 can be configured such that the transverse deformation resistance of the first region 2100 is different from the transverse deformation resistance of the second region 2200.

For example, with reference now to FIGS. 2, 4, and 4A the second region 2200 of the support structure 2000 is comprised of a plurality of frame members including one or more strut elements 2202. The strut elements 2102 may be coupled together or may be configured to interface at one or more interface regions 2204.

In some embodiments, the plurality of strut elements 2202 define a collapsible (e.g., elastically) and expandable (e.g., self-expanding, or expandable via balloon or other suitable mechanism as discussed herein) framework, and also serve to support one or more leaflets, as mentioned above. As shown, the plurality of strut elements 2202 define a plurality of rows of closed cells 2218 (e.g., row 2206 and row 2208) defining an undulating, alternating pattern of proximal-facing apices 2210 pointing in a proximal direction and distal-facing apices 2212 pointing in a distal direction. As shown in FIG. 4, there are fifteen rows of closed cells 2218, although greater or fewer numbers are contemplated including odd and even numbers of rows (e.g., 1, 2, 4, 12, 20).

In various examples, each of the apex angles 2214 of each of the distal-facing apices 2210 has a value that is approximately the same in more than one of the plurality of rows (e.g., 2206 and 2208) of closed cells 2218. For example, in some embodiments, each the apex angles 2214 is within 10% of a common apex angle defined by the plurality of rows of distal-facing apices 2210. In other embodiments, each of the apex angles is within 5%, 15%, 20%, or some other value of a common apex angle. In some examples, the common apex angle is 30 degrees, although any of a variety of common apex angles is contemplated (e.g., 10, 15, 20, 30, 40, 45, 50, 60, 90 degrees and ranges between any of those vales).

In various examples, each of the apex angles 2216 of each of the proximal-facing apices 2212 has a value that is approximately the same in more than one of the plurality of rows (e.g., 2206 and 2208) of closed cells 2218. For example, in some embodiments, each the apex angles 2216 is within 10% of a common apex angle defined by the plurality of rows of proximal-facing apices 2212. In other embodiments, each of the apex angles is within 5%, 15%, 20%, or some other value of a common apex angle. In some examples, the common apex angle is 30 degrees, although any of a variety of common apex angles is contemplated (e.g., 10, 15, 20, 30, 40, 45, 50, 60, 90 degrees and ranges between any of those vales).

In some examples, the apex angles 2214 and/or the apex angles 2216 of one or more columns of closed cells 2218 defined by the plurality of closed cells 2218 are approximately the same as another one of the columns of closed cells 2218. For example, the apex angles of one or more columns is optionally within 10% of a common apex angle defined by the one or more columns of closed cells 2218 of proximal-facing apices 2212 and/or distal-facing apices 2210. In other embodiments, each of the apex angles is within 5%, 15%, 20%, or some other value of a common apex angle. In some examples, the common apex angle is 30 degrees, although any of a variety of common apex angles is contemplated (e.g., 10, 15, 20, 30, 40, 45, 50, 60, 90 degrees and ranges between any of those values).

The closed cells 2218 of the second region 2200 of the support structure 2000 generally intersect with one another at intersection locations Pp. As shown in the example of FIG. 4, each closed cell 2218 of the second region 2200 of the support structure 2000 has a cell height 2220 a cell width 2222, wherein the cell width 2222 can be generally understood to be perpendicular to the cell height 2220. Moreover, each closed cell 2218 has a first lateral-facing apex 2224 defining an apex angle 2228 and a second lateral facing-apex 2226 opposite the first lateral-facing apex 2224 and defining an apex angle 2230.

In a similar manner to the apex angles 2214 and the apex angles 2216, in various examples, each of the apex angles 2228 and/or apex angles 2230 of has approximately the same value between one or more of the plurality of closed cells 2218 (e.g., within 10% of a common apex angle, although other values such as values within 5%, 15%, 20%, or some other value of a common apex angle are contemplated). In some examples, the common apex angle is 30 degrees, although any of a variety of common apex angles is contemplated (e.g., 10, 15, 20, 30, 40, 45, 50, 60, 90 degrees and ranges between any of those values).

In various examples, the cell heights 2220 of the closed cells 2218 may be the same or different for different rows and/or columns of closed cells 2218 of the second region 2200 of the support structure 2000. Similarly, the cell widths 2222 of the closed cells 2218 may be the same or different for different rows and/or columns of closed cells 2218 of the second region 2200 of the support structure 2000.

In some embodiments, varying the apex angles of the proximal and distal facing apices 2210 and 2212 and the lateral-facing apices 2224 and 2226 of the closed cells helps control compaction forces (e.g., the force needed for diametrically compacting the prosthetic valve 1000 to a compact delivery configuration) as well as the transverse deformation resistance of the particular region of the support structure 2000 upon deployment. Thus, in various examples, the structural support 2000 can be configured such that the transverse deformation resistance of the second region 2200 is different from the transverse deformation resistance of the second region 2200.

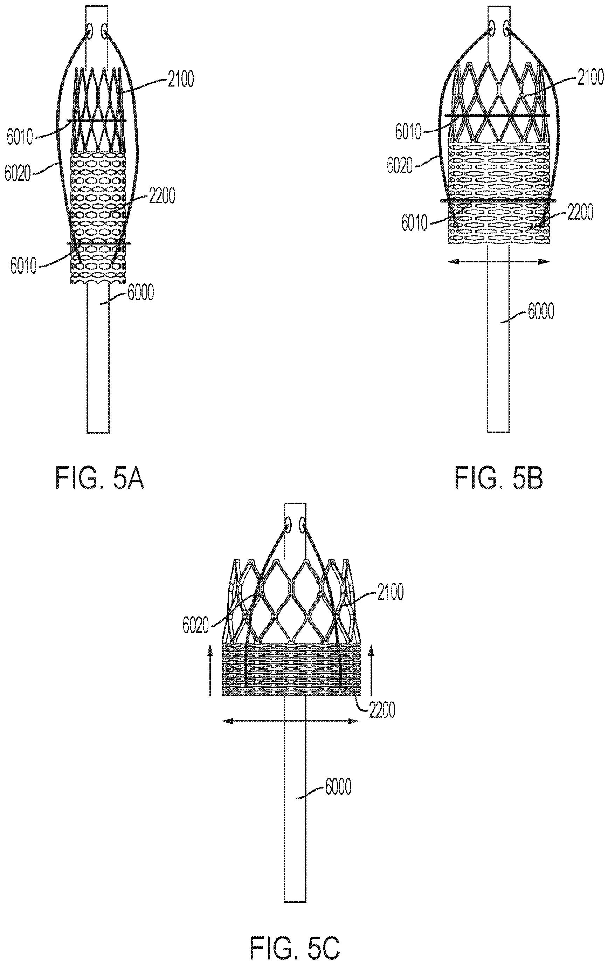

FIGS. 5A to 5C are schematic views showing the support structure 2000 through a deployment sequence using a delivery system 6000, according to some examples. The delivery system 6000 optionally includes circumferential constraints 6010 for retaining the support structure 2000 in a diametrically compacted state and longitudinal constraints 6020 for applying a longitudinal compression force on the second region 2200. FIG. 5A shows the support structure 2000 in an initial state including the first region 2100 and the second region 2200 being maintained at a compacted, delivery diameter, or profile by the delivery system 6000. FIG. 5B shows the support structure 2000 in an intermediate state, with the first region 2100 and the second region 2200 diametrically expanded to an intermediate diameter, or profile. In some examples, the support structure 2000 is configured to self-expand to the intermediate state upon release of the diametric constraints 6010. FIG. 5C shows the support structure 2000 in a deployed state, with the support structure, including the first region 2100 and the second region 2200, diametrically expanded to a deployed diameter, or profile. As shown in FIG. 5C, the second region has been longitudinally compacted. In some examples, a longitudinal compression force is applied to the second region 2200. For example, a longitudinal compression force may be applied via the longitudinal constraints 6020 associated with the delivery system 6000. In some examples not according to the invention, the second region 2200 may include features for locking the second region 2200 in the longitudinally compressed state.

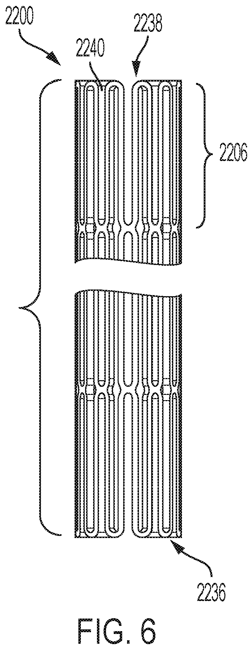

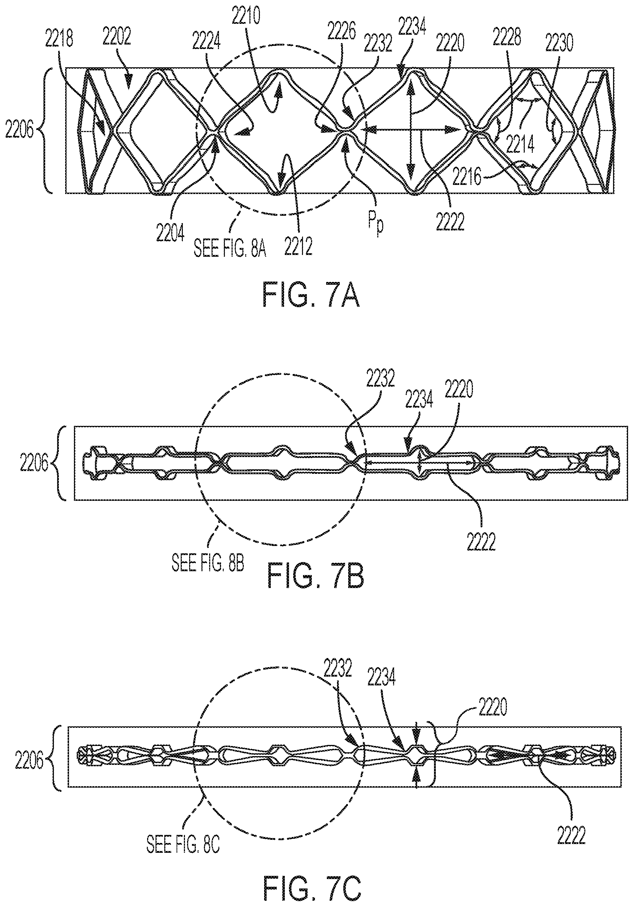

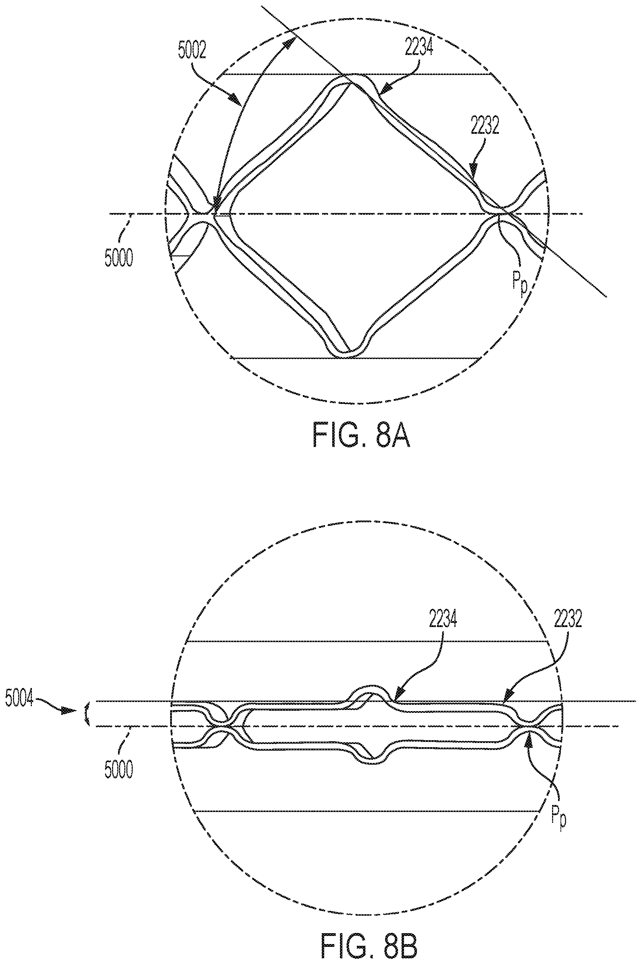

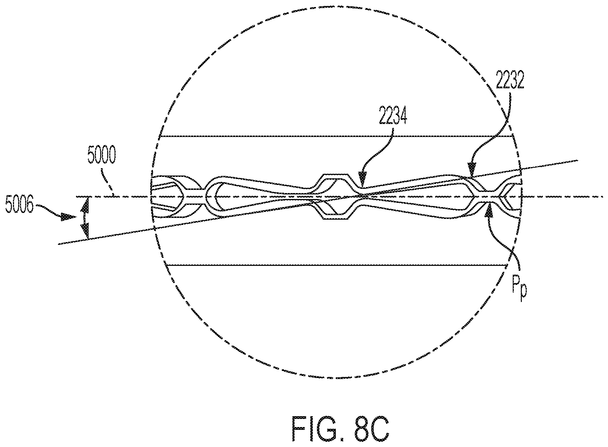

During longitudinal compression, the strut elements 2202 are deformed, which includes modifying the various apex angles of the second region 2200. FIG. 6 shows rows of closed cells of the second region 2200 in the initial state, including row 2206 which is representative of the remaining rows of the second region 2200, according to some examples. FIGS.7A to 7C show row 2206 as it transitions from the intermediate state to the deployed state, including modification of the apex angles of the proximal and distal facing apices 2210 and 2212 and the lateral-facing apices 2224 and 2226 of the closed cells during transition of the second region from the intermediate state to the deployed state. FIGS. 8A to 8C show a single closed cell of the row 2206 in greater detail. In some examples, the second region 2200 self-expands from the initial state (e.g., FIG. 6) to the intermediate state (e.g., FIG. 7A and 8A) and is longitudinally compressed to the deployed state (FIG. 7C and 8C). As shown in various figures, the apex angles of the proximal and distal facing apices 2210 and 2212 and the lateral-facing apices 2224 and 2226 of the closed cells 2218 are generally equal, although varying configurations are also contemplated.

As shown in FIGS. 7A to 7C, in the intermediate state of FIG. 7A and 8A, the height 2220 of the closed cells 2218 of row 2206 has been reduced relative to the initial state of FIG. 6 (while the width 2222 has been increased) such that the apex angles 2214 and 2216 of the proximal and distal facing apices 2210 and 2212 have been increased relative to those shown in FIG. 6, and such that the apex angles 2214 and 2216 of the lateral-facing apices 2224 and 2226 of the closed cells 2218 of row 2206 have decreased relative to those shown in FIG. 6. It is to be appreciated that the apex angles referred to herein may be defined as a relative angle between adjacent strut elements 2202 (such as between the longitudinal axes of adjacent strut elements) intersecting at point Pp.

As shown in FIGS. 7B and 7C (and FIGS. 8B and 8C), the height 2220 of the closed cells 2218 of row 2206 has been further reduced (while the width 2222 has been further increased) during transition to the deployed state such that the apex angles 2214 and 2216 of the proximal and distal facing apices 2210 and 2212 have been increased relative to those shown in FIGS. 6 and 7A, and such that the apex angles 2228 and 2230 of the lateral-facing apices 2224 and 2226 of the closed cells 2218 of row 2206 have decreased relative to those shown in FIGS. 6 and 7A. In various embodiments, as the height 2220 is further reduced, the angles 2214 and 2216 of the proximal and distal facing apices 2210 and 2212 approaches (and in some instances breaches) one hundred eighty (180) degrees (also referred to as centering). For example, the configuration shown in FIGS. 7B and 8B illustrates the angles 2214 and 2216 of the proximal and distal facing apices 2210 and 2212 at approximately one hundred eighty (180) degrees, while the configuration shown in FIGS. 7C and 8C illustrates the angles 2214 and 2216 of the proximal and distal facing apices 2210 and 2212 at an angle exceeding one hundred eighty (180) degrees (also referred to herein as overcentering).

Increasing the apex angles 2214 and 2216 of the proximal and distal facing apices 2210 and 2212 (e.g., increasing the angle between adjacent strut elements 2202), operates to increase the transverse deformation resistance of the row 2206 of closed cells 2218, thereby increasing the transverse deformation resistance of the region of the structural support 2000 where the row 2206 of closed cells 2218 is located (e.g., in this case, the second region 2200). It should also be understood that other factors, including material selection, strut geometry (cross-section and curvature), and other characteristics may be selected to vary transverse deformation resistance of the second region 2200.

In various examples, the intersection points (e.g., Pd and Pp) defines a hinge region for the various strut elements (e.g., 2102 and 2202) intersecting or otherwise interfacing with one another at the intersection points (e.g., Pd and Pp). In some examples, the strut elements (e.g., 2102 and/or 2202) of the support structure 2000 may include one or more sections thereof that are configured to deform to facilitate or accommodate the change in apex angles discussed above. These deformations of the one or more sections of the strut elements may include elastic deformation, plastic deformation, or combinations thereof. For instance, as shown in FIGS. 7A to 8C, the strut elements 2202 include regions of reduced cross-sectional area 2232 and 2234 that are configured to deform to facilitate or accommodate the change in apex angles discussed above. It is to be appreciate that the deformations of the one or more sections of the strut elements may be achieved by additionally or alternatively reducing the stiffness (e.g., by reduced elastic modulus or hardness) of the deformable section.

Referring back now to FIG. 4, in various examples, the second region 2200 includes a proximal end region 2236 and a distal end region 2238. In various examples, the distal end region 2238 includes a distal edge 2240 that is coupled to a proximal edge 2136 (see FIG. 2) of the first region 2100. The first and second regions 2100 and 2200 are optionally coupled by being integrally formed (e.g., during an etching or cutting operation) or otherwise attached (e.g., by welding, adhesives, or other means). In some examples, the distal edge 2240 of the second region 2200 is defined, at least in part, by the distal most apices of closed cells 2218 of the second region 2200, and the proximal edge 2136 of the first region 2100 is defined, at least in part, by the proximal most apices of the closed cells 2118 of the first region 2100.

In various examples, decreasing the height 2220 of the closed cells 2218 the second region 2200 may be accomplished by drawing together the proximal and distal end regions 2236 and 2238 of the second region 2200 of the support structure 2200. For instance, in a delivery configuration, the proximal and distal end regions 2236 and 2238 may be a first distance apart from one another (see FIG. 6), and upon deployment of the prosthetic valve 1000, the proximal and distal ends 2236 and 2238 of the second region 2200 of the support structure 2000 are drawn together such that the proximal and distal end regions 2236 and 2238 are closer in proximity in the deployed configuration (FIGS. 2, 5C, 7C, and 8C) than they are in the delivery configuration (FIGS. 5A and 6).

In some examples, like that shown in FIG. 6, in the delivery configuration, the height 2220 of the closed cells 2218 is increased or even maximized (while the width 2222 is decreased or even minimized) such that the apex angles 2214 and 2216 of the proximal and distal facing apices 2210 and 2212 has been decreased relative to those shown in FIGS. 2, 5C, 7C, and 8C, and such that the apex angles 2228 and 2230 of the lateral-facing apices 2224 and 2226 of the closed cells 2218 have increased relative to those shown in FIG. 4.

In some examples, drawing together the proximal and distal end regions 2236 and 2238 of the second region 2200 of the support structure during or following deployment to an enlarged diameter has the further effect of increasing the radial dimension of the second region from a first diameter to a second larger diameter. For example, the diameter of the second region 2200 shown in FIG. 2 is greater than the diameter of the second regions 2200 shown in FIG. 6, while the distance between the proximal and distal end regions 2236 and 2238 shown in FIG. 2 is less than the distance between the proximal and distal end regions 2236 and 2238 shown in FIG. 6. As discussed above, as the height 2220 of the closed cells 2218 is reduced and the width 2222 is increased, the apex angles 2214 and 2216 of the proximal and distal facing apices 2210 and 2212 increases and the apex angles 2228 and 2230 of the lateral-facing apices 2224 and 2226 of the closed cells 2218 decreases. It should also be appreciated that, some examples, as the height 2220 of the closed cells 2218 is reduced and the width 2222 is increased, a curvature of the deformation sections (e.g., sections 2232 and 2234) of the struts (e.g., struts 2202) increases. Such an increase in curvature is shown in comparison of FIGS. 5A to 7A. As shown, a curvature of the deformation sections 2232 and 2234 is increased (i.e., more curved) in FIG. 6A relative to FIG. 5A. Similarly, as shown, a curvature of the deformation sections 2232 and 2234 is increased (i.e., more curved) in FIG. 7A relative to FIG. 6A. Likewise, it should also be appreciated that, in some examples, as the height 2220 of the closed cells 2218 is reduced and the width 2222 is increased, a relative angle between the strut elements 2202 and a transverse datum 5000 extending along the width profile of a closed cell 2218 between lateral-facing apices transitions from a non-parallel positive angle 5002, to a zero angle 5004 (e.g., parallel relationship), and to a non-parallel negative angle 5006.

In some examples, the deformation regions (e.g., 2232 and 2234) of the strut elements (e.g., 2102 and 2202) act as live hinges in that these deformation regions permit the strut elements to pivot or change angle relative to the intersections regions (e.g., Pd and Pp) as well as permitting the strut elements to change angle relative to one another. In some examples, one or more of the first and second regions 2100 and 2200 of the support structure 2000 are formed by laser cutting a tube, such as a nitinol tube. In various examples, the second region 2200 (and optionally the first region 2100) are shape set at the intermediate state, such that the second region 2200 is configured to self-expand toward the intermediate state at the intermediate diameter, or profile. According to one example, support structure 2000 is cut from a 6mm outer diameter nitinol tube and shape set to a 24mm outer diameter. The support structure 2000 is crushed from 24mm to a 6mm delivery diameter. The final deployment diameter of the support structure 2000 may be 29mm or more. These dimensions are meant as examples only, and a variety of shapes and sizes are contemplated.

In some examples, one or more of the first and second regions 2100 and 2200 of the support structure may be formed such that the intersection points (e.g., Pd and Pp) define an intersection hub to which the strut elements are coupled. For instance, in some examples, one or more of the strut elements forming a respective one of the first and second regions 2100 and 2200 of the support structure 2000 are coupled to the hub at a reduced cross-section region (e.g., the end sections) in order to reduce strain on the strut elements, as the strut elements are operable to pivot about the hubs without requiring substantial deformation of the more central sections of the strut elements. This feature may be particularly helpful during longitudinal compression, and such features may also help to minimize or reduce elastic recoil back from the longitudinally compressed configuration due to energy stored in the strut elements due longitudinal compression of the second region 2200.

It is also to be appreciated that the support structure 2000 may be initially shape set to any of the configurations discussed herein, including the delivery configuration, the deployed configuration, or any configuration therebetween (e.g., a partially deployed configuration). For instance, in some examples, the support structure 2000 may be shape set to a partially deployed configuration wherein the prosthetic valve 1000 is crushed down to a delivery configuration (e.g., stored potential energy), and wherein the fully deployed configuration requires mechanically expanding the prosthetic valve 1000 to beyond the shape set configuration. In some examples, mechanically expanding the prosthetic valve 1000 to beyond the shape set configuration requires one or more of radially expanding the first region 2100 and radially expanding and axially compressing the second regions 2200. In some examples, radially expanding and axially compressing the second regions 2200 requires mechanical intervention, such as one or more axial tensioning screws that engage with one or more portions of the prosthetic valve to draw the proximal and distal end regions 2236 and 2238 of the support structure 2000 closer together that they are in the delivery configuration. Additionally, or alternatively, mechanical intervention may include utilizing one or more suturing elements or tethers to draw together the proximal and distal end regions 2236 and 2238 of the support structure. In some examples, the mechanical intervention means discussed above may remain in place to lock the support structure 2000 (and thus the prosthetic valve 1000) in the fully deployed configuration).

In various examples not according to the invention, the prosthetic valve 1000 includes a locking mechanism for securing the second region in an axially, or longitudinally compressed state. The locking mechanism may include components secured to the second region 2200 of the support structure 2000 (e.g., integrally formed therewith) that interact with one another during collapse of the cells formed by the second region 2200 of the support structure 2000. The locking mechanism associated with a cell or set of cells can then lock and retain the corresponding cell(s) in a collapsed configuration.

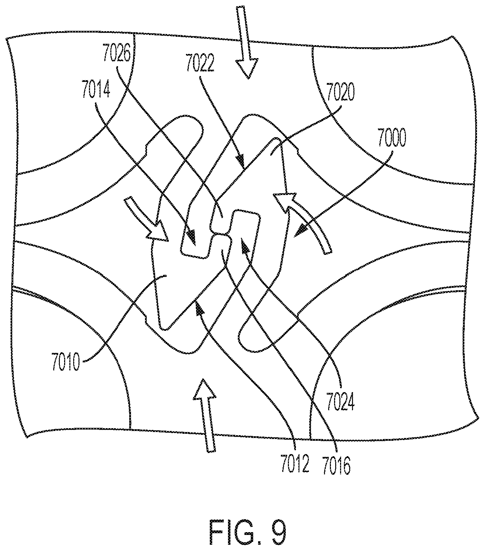

FIG. 9 illustrates a locking mechanism 7000 that may be incorporated into one or more cells of one or more rows of the second region 2200 for longitudinally locking the second region 2200 in the longitudinally compressed state. As shown, the locking mechanism 7000 includes a first lock component 7010 and a second lock component 7020. The first and second lock components 7010 and 7020 each optionally project inwardly from opposite apices in a cell. The first lock component 7010 includes a first sliding surface 7012 and the second lock component 7020 includes a second sliding surface 7022. The two components 7010 and 7020 slide against each other during longitudinal compression of the second region 2200 and elastically deflect outwardly away from one another.

As shown, the first and second lock components 7010 and 7020 form a first receiver 7014 and a second receiver 7024, respectively. And, as shown, the first and second lock components 7010 and 7020 include a first projection 7016 and a second projection 7026, respectively. Once the first and second lock components 7010 and 7020 have slid far enough, the first and second projections 7016 and 7026 slide past one another such that the first and second lock components 7010 and 7020 are able to move back toward one another, with the first projection 7016 sliding into the first receiver 7014 and the second projection 7026 sliding into the second receiver 7024, thereby locking the first and second lock components 7010 and 7020 together and the associated cell in a longitudinally compressed state. As previously referenced, any number of locking mechanisms 7000 may be incorporated into the second region 2200 to facilitate locking the various rows of closed cells in a more closed, or longitudinally compressed state.

Although the locking mechanism 7000 is shown and described in association with a prosthetic valve, it should be understood that the locking mechanism can be applied to any of a variety of expanding and expandable support structures, including any expandable (e.g., self-expanding) tubular framework defining a pattern of cells.

### Leaflet Materials

In various examples, the leaflet or leaflet construct is formed of a biocompatible, synthetic material (e.g., including ePTFE and ePTFE composites, or other materials as desired). In other examples, the leaflet construct 104 is formed of a natural material, such as repurposed tissue, including bovine tissue, porcine tissue, or the like.

As used herein, the term "elastomer" refers to a polymer or a mixture of polymers that has the ability to be stretched to at least 1.3 times its original length and to retract rapidly to approximately its original length when released. The term "elastomeric material" refers to a polymer or a mixture of polymers that displays stretch and recovery properties similar to an elastomer, although not necessarily to the same degree of stretch and/or recovery. The term "non-elastomeric material" refers to a polymer or a mixture of polymers that displays stretch and recovery properties not similar to either an elastomer or elastomeric material, that is, considered not an elastomer or elastomeric material.

In accordance with some embodiments herein, the leaflet comprises a composite material having at least one porous synthetic polymer membrane layer having a plurality of pores and/or spaces and an elastomer and/or an elastomeric material and/or a non-elastomeric material filling the pores and/or spaces of the at least one synthetic polymer membrane layer. In accordance with other examples, the leaflet further comprises a layer of an elastomer and/or an elastomeric material and/or a non-elastomeric material on the composite material. In accordance with examples, the composite material comprises porous synthetic polymer membrane by weight in a range of about 10% to 90%.

An example of a porous synthetic polymer membrane includes expanded fluoropolymer membrane having a node and fibril structure defining the pores and/or spaces. In some examples, the expanded fluoropolymer membrane is expanded polytetrafluoroethylene (ePTFE) membrane. Another example of porous synthetic polymer membrane includes microporous polyethylene membrane.

Examples of an elastomer and/or an elastomeric material and/or a non-elastomeric material include, but are not limited to, copolymers of tetrafluoroethylene and perfluoromethyl vinyl ether (TFE/PMVE copolymer), (per)fluoroalkylvinylethers (PAVE), urethanes, silicones (organopolysiloxanes), copolymers of silicon-urethane, styrene/isobutylene copolymers, polyisobutylene, polyethylene-co-poly(vinyl acetate), polyester copolymers, nylon copolymers, fluorinated hydrocarbon polymers and copolymers or mixtures of each of the foregoing. In some examples, the TFE/PMVE copolymer is an elastomer comprising essentially of between 60 and 20 weight percent tetrafluoroethylene and respectively between 40 and 80 weight percent perfluoromethyl vinyl ether. In some examples, the TFE/PMVE copolymer is an elastomeric material comprising essentially of between 67 and 61 weight percent tetrafluoroethylene and respectively between 33 and 39 weight percent perfluoromethyl vinyl ether. In some examples, the TFE/PMVE copolymer is a non-elastomeric material comprising essentially of between 73 and 68 weight percent tetrafluoroethylene and respectively between 27 and 32 weight percent perfluoromethyl vinyl ether. The TFE and PMVE components of the TFE-PMVE copolymer are presented in wt%. For reference, the wt% of PMVE of 40, 33-39, and 27-32 corresponds to a mol% of 29, 23-28, and 18-22, respectively.

In some examples, the TFE-PMVE copolymer exhibits elastomer, elastomeric, and/or non-elastomeric properties.

In some examples, the composite material further comprises a layer or coating of TFE-PMVE copolymer comprising from about 73 to about 68 weight percent tetrafluoroethylene and respectively from about 27 to about 32 weight percent perfluoromethyl vinyl ether.

In some examples, the leaflet is an expanded polytetrafluoroethylene (ePTFE) membrane having been imbibed with TFE-PMVE copolymer comprising from about 60 to about 20 weight percent tetrafluoroethylene and respectively from about 40 to about 80 weight percent perfluoromethyl vinyl ether, the leaflet further including a coating of TFE-PMVE copolymer comprising from about 73 to about 68 weight percent tetrafluoroethylene and respectively about 27 to about 32 weight percent perfluoromethyl vinyl ether on the blood-contacting surfaces.

As discussed above, the elastomer and/or an elastomeric material and/or a non-elastomeric material may be combined with the expanded fluoropolymer membrane such that the elastomer and/or the elastomeric material and/or the non-elastomeric material occupies substantially all of the void space or pores within the expanded fluoropolymer membrane.

Some examples of suitable leaflet materials may be found in U.S. Patent 8,961,599 to Bruchman et al. ("Durable High Strength Polymer Composite Suitable for Implant and Articles Produced Therefrom"); U.S. Patent 8,945,212 to Bruchman et al. ("Durable Multi-Layer High Strength Polymer Composite Suitable for Implant and Articles Produced Therefrom"); U.S. 9,554,900 to Bruchman et al. ("Durable High Strength Polymer Composites Suitable for Implant and Articles Produced Therefrom"); and U.S. Pat. App. Pub. 2015/0224231 to Bruchman et al. ("Coherent Single Layer High Strength Synthetic Polymer Composites for Prosthetic Valves").

### Frame Materials

The frames can be etched, cut, laser cut, stamped, three-dimensional printed or wire wound, among other suitable processes. The frames can be self-expanding or balloon expandable (e.g., when configured for transcatheter implantation) or non-expandable (e.g., when configured for surgical implantation). The various frames can comprise materials, such as, but not limited to, any metallic or polymeric material, such as an elastically (e.g., nitinol) or plastically (e.g., stainless steel) deformable metallic or polymeric material that is generally biocompatible. Other materials suitable for any of the frames described herein include, but are not limited to, other titanium alloys, stainless steel, cobalt-nickel alloy, polypropylene, acetyl homopolymer, acetyl copolymer, a drawn filled tube (e.g., nitinol wire with a platinum core), other alloys or polymers, or any other material that is generally biocompatible having adequate physical and mechanical properties to function as a frame as described herein.

### Methods of Making

Various methods of making prosthetic valves are contemplated for the various prosthetic valves described herein. Generally, the methods include providing a frame and a leaflet construct according to any of the above-described embodiments and securing the leaflet construct to the frame.

In some methods of making prosthetic valves, the leaflet construct is at least partially coupled to the frame by a looped structure. For example, in some methods the commissure tabs of the leaflet construct define one or more loops that are passed through slots in the commissure posts of the frames, such as the commissure posts according to any of the frame embodiments previously described. In some examples, inner retaining elements pass through one or more of the loops to help widen the loops and help prevent the loop(s), or passes of material, from pulling outwardly through the slots in the commissure posts. Outer retaining elements additionally or alternatively help prevent the loop(s), or passes of material, from pulling inwardly through the slots in the commissure posts. In various examples, the loop(s) of material are optionally coupled to one another and/or to the frame (e.g., bonded or adhered by an outer wrap of film, sutured, or otherwise secured) to help secure the commissure tabs to the commissure posts. In various examples, the body portions of the leaflets are optionally attached to the frame using attachment tabs secured through and folded over the outer side of the frame and/or cover. In some methods, leaflet retention features are coupled onto (e.g., slidingly received onto) leaflet frame projections to secure the leaflets to the frame. These and other methods should be apparent from the foregoing disclosure.

## Claims

1. A support structure (2000) for a prosthetic heart valve (1000), the support structure (2000) including a tubular body having a longitudinal axis (X_{f}), the tubular body comprising:
a first region (2100) that is annular shape and is **characterized by** a first transverse deformation resistance; and
a second region (2200) that is annular in shape and is **characterized by** a second transverse deformation resistance, the second region (2200) including a plurality of strut elements (2202) defining the annular shape of the second region (2200), at least a portion of each of the plurality of strut elements (2202) having a region of reduced cross section including a width in a radial direction transverse to the longitudinal axis (X_{f}) of the tubular body and a thickness in a longitudinal direction parallel to the longitudinal axis (X_{f}) of the tubular body, the width in the region of reduced cross section being at least four times the thickness such that the second region exhibits a relatively higher degree of compressibility in the longitudinal direction than in the radial direction.

2. The support structure (2000) of claim 1, wherein the second transverse deformation resistance is greater than the first transverse deformation resistance.

3. The support structure (2000) of claim 1, wherein the first region (2100) includes a plurality of strut elements (2102) defining the annular shape of the first region (2100), and further wherein at least a portion of each of the plurality of strut elements (2102) of the first region (2100) has a width in the radial direction transverse to the longitudinal axis (X_{f}) of the tubular body and a thickness in the longitudinal direction parallel to the longitudinal axis (X_{f}) of the tubular body, the width of each of the plurality of strut elements (2102) of the first region being less than 4 times the thickness of each of the plurality of strut elements (2102) of the first region.

4. The support structure (2000) of claim 1, further including one or more leaflets (3000) coupled to the first region (2100); and optionally
wherein the one or more leaflets (3000) are formed of a natural material; or
wherein the one or more leaflets (3000) are formed of a synthetic material.

## Patentansprüche

1. Trägerstruktur (2000) für eine Herzklappenprothese (1000), wobei die Trägerstruktur (2000) einen röhrenförmigen Körper mit einer Längsachse (Xε) beinhaltet, wobei der röhrenförmige Körper umfasst:
eine erste Region (2100), die eine Ringform ist und durch einen ersten Querverformungswiderstand gekennzeichnet ist; und
eine zweite Region (2200), die ringförmig ist und durch einen zweiten Querverformungswiderstand gekennzeichnet ist, wobei die zweite Region (2200) eine Vielzahl von Strebenelementen (2202) beinhaltet, die die Ringform der zweiten Region (2200) definieren, wobei mindestens ein Teil jedes der Vielzahl von Strebenelementen (2202) eine Region mit verringertem Querschnitt aufweist, der eine Breite in einer Radialrichtung quer zur Längsachse (Xε) des röhrenförmigen Körpers und eine Dicke in einer Längsrichtung parallel zur Längsachse (Xε) des rohrförmigen Körpers beinhaltet, wobei die Breite in der Region verringerten Querschnitts mindestens das Vierfache der Dicke beträgt, sodass die zweite Region in der Längsrichtung einen relativ höheren Grad an Kompressibilität zeigt als in der Radialrichtung.

2. Trägerstruktur (2000) nach Anspruch 1, wobei der zweite Querverformungswiderstand größer ist als der erste Querverformungswiderstand.

3. Trägerstruktur (2000) nach Anspruch 1, wobei die erste Region (2100) eine Vielzahl von Strebenelementen (2102) beinhaltet, die die Ringform der ersten Region (2100) definieren, und ferner wobei mindestens ein Teil jedes der Vielzahl von Strebenelementen (2102) der ersten Region (2100) eine Breite in der Radialrichtung quer zur Längsachse (Xε) des rohrförmigen Körpers und eine Dicke in der Längsrichtung parallel zur Längsachse (Xε) des rohrförmigen Körpers aufweist, wobei die Breite jedes der Vielzahl von Strebenelementen (2102) der ersten Region weniger als das Vierfache der Dicke jedes der Vielzahl von Strebenelementen (2102) der ersten Region beträgt.

4. Trägerstruktur (2000) nach Anspruch 1, ferner beinhaltend ein oder mehrere Segel (3000), die mit der ersten Region (2100) gekoppelt sind; und optional
wobei das eine oder die mehreren Segel (3000) aus einem natürlichen Material gebildet sind; oder
wobei das eine oder die mehreren Segel (3000) aus einem synthetischen Material gebildet sind.

## Revendications

1. Structure de support (2000) pour une valvule cardiaque prothétique (1000), la structure de support (2000) comprenant un corps tubulaire comportant un axe longitudinal (X_{f}), le corps tubulaire comprenant :
une première région (2100) qui est de forme annulaire et est **caractérisée par** une première résistance à la déformation transversale ; et
une seconde région (2200) qui est de forme annulaire et est **caractérisée par** une seconde résistance à la déformation transversale, la seconde région (2200) comprenant une pluralité d'éléments entretoises (2202) définissant la forme annulaire de la seconde région (2200), au moins une partie de chacun de la pluralité d'éléments entretoises (2202) comportant une région de section transversale réduite comprenant une largeur dans une direction radiale transversale à l'axe longitudinal (Xε) du corps tubulaire et une épaisseur dans une direction longitudinale parallèle à l'axe longitudinal (Xf) du corps tubulaire, la largeur dans la région de section transversale réduite étant au moins quatre fois supérieure à l'épaisseur de sorte que la seconde région présente un degré de compressibilité relativement plus élevé dans la direction longitudinale que dans la direction radiale.

2. Structure de support (2000) de la revendication 1, ladite seconde résistance à la déformation transversale étant supérieure à la première résistance à la déformation transversale.

3. Structure de support (2000) de la revendication 1, ladite première région (2100) comprenant une pluralité d'éléments entretoises (2102) définissant la forme annulaire de la première région (2100), et en outre au moins une partie de chacun de la pluralité d'éléments entretoises (2102) de la première région (2100) comportant une largeur dans la direction radiale transversale à l'axe longitudinal (Xε) du corps tubulaire et une épaisseur dans la direction longitudinale parallèle à l'axe longitudinal (Xε) du corps tubulaire, la largeur de chacun de la pluralité d'éléments entretoises (2102) de la première région étant inférieure à 4 fois l'épaisseur de chacun de la pluralité d'éléments entretoises (2102) de la première région.

4. Structure de support (2000) de la revendication 1, comprenant en outre un ou plusieurs feuillets (3000) couplés à la première région (2100) ; et éventuellement
ledit ou lesdits feuillets (3000) étant formés d'un matériau naturel ; ou ledit ou lesdits feuillets (3000) étant formés d'un matériau synthétique.
